# EUROPEAN PATENT APPLICATION

(11) **EP 4 431 487 A1**
(43) Date of publication of application: **18.09.2024**
(21) Application number: 23162609.4
(22) Date of filing: 17.03.2023
(51) Int. Cl.: C07C 51/00, C07C 53/02, C07C 53/08

(54) **METHOD FOR OBTAINING AN ALKANOIC ACID**

(71) Applicant: OxFA GmbH, 96110 Scheßlitz (DE)
(72) Inventor: SCHMIDT, Matthias, 91362 Pretzfeld (DE); SCHOLZ, Gunthard, 96163 Gundelsheim (DE); KOHLER, Florian, 90419 Nürnberg (DE)
(74) Representative: Dr. Gassner & Partner mbB

(57) **Abstract**

The invention relates to a method for obtaining an alkanoic acid, wherein molecular oxygen, a solid biogenic material and a solution of a catalyst are used in a catalytic reaction, wherein the catalyst is a polyoxometallate ion, a VO²⁺ containing salt, a [VO₃]⁻ containing salt, a vanadium phosphate, a vanadium pentoxide, an ammonium iron sulfate, a copper sulfate or a nickel sulfate, wherein the biogenic material is insoluble in the solution of the catalyst, wherein the method comprises providing the molecular oxygen, the biogenic material and the solution of the catalyst and forming a triphasic composition by bringing into contact the biogenic material, the solution of the catalyst and the molecular oxygen.

## Description

The invention relates to a method for obtaining an alkanoic acid, wherein molecular oxygen, a biogenic material and a solution of a catalyst are used in a catalytic reaction.

WO 2012/034839 A1 discloses a method for catalytically producing formic acid. A polyoxometallate ion serving as catalyst and of the general formula [PMoₓV_{y}O₄₀]⁵⁻ is contacted at a temperature below 120 °C with an alpha-hydroxyaldehyde, an alpha-hydroxycarboxylic acid, a carbohydrate, or a glycoside in a liquid solution, where 6 < x < 11 and 1 < y < 6, and x + y = 12, with x and y each being an integer.

In WO 2016/116405 A1, a method for separating formic acid from a reaction mixture comprising in addition to formic acid a polyoxometallate ion of the general formula [PMoₓV_{y}O₄₀]ⁿ⁻, where 6 ≤ x ≤ 11, 1 ≤ y ≤ 6, x + y = 12 and 3 < n < 10, where n, x and y are each integers, is disclosed. The separation is accomplished by extraction by means of a linear primary alcohol. The carbon chain of the alcohol has five to twelve carbon atoms, and the reaction mixture is in a protic solvent.

EP 3 484 846 A1 discloses a method for catalytically producing formic acid and regenerating the catalyst employed. A vanadyl ion, vanadate ion or polyoxometallate ion of the general formula [PMoₓV_{y}O₄₀]ⁿ⁻ serving as catalyst is contacted at a temperature above 70 °C and below 160 °C with an alpha-hydroxyaldehyde, an alpha-hydroxycarboxylic acid, a carbohydrate, a glycoside or a polymer containing a carbon chain and having at least one OH group bonded repeatedly as substituent to the carbon chain and/or having an O, N or S atom present repeatedly in the carbon chain, in a liquid solution in a vessel, where 6 ≤ x ≤ 11 and 1 ≤ y ≤ 6 and 3 < n < 10 and x + y = 12, where n, x and y are each an integer. The reduced catalyst is returned by oxidation to its original state. For this purpose the solution is contacted with a gas comprising a volume fraction of at least 18 % of oxygen at a pressure of at least 2 bar and at most 16 bar, by means of a mixing apparatus or via a liquid-impermeable, gas-permeable membrane, where CO and/or CO₂ formed in the reaction and passing into the gas are/is taken off in a quantity such that the volume fraction of CO and CO₂ together in the gas does not exceed 80 %.

In WO 2016/120169 A1, a method for the catalytic generation of formic acid at an oxygen partial pressure below 1 bar and regeneration of the catalyst used therefor is disclosed. A polyoxometallate ion, serving as a catalyst, of general formula [PMoₓV_{y}O₄₀]ⁿ⁻ is brought into contact, at a temperature above 70 °C and below 120 °C, with an alpha-hydroxyaldehyde, an alpha-hydroxycarboxylic acid, a carbohydrate, a glycoside or a polymer containing a carbon chain and having at least one OH group bonded to the carbon chain repeatedly as a substituent and/or having an O, N or S atom contained repeatedly in said carbon chain, in a liquid solution in a vessel, wherein 6 ≤ x ≤ 11 and 1 ≤ y ≤ 6 and 3 < n < 10 and x + y = 12, wherein each of n, x and y is an integer. The catalyst reduced in this way is returned to its starting state by oxidation. The contacting is carried out in the vessel at an oxygen partial pressure below 1 bar. For oxidizing the catalyst, a portion of the liquid solution is diverted out of the vessel, has oxygen or a gas mixture containing oxygen applied thereto at an oxygen partial pressure of 1 to 500 bar, and is subsequently fed back to the rest of the liquid solution. The oxygen partial pressure in the vessel is constantly maintained below 1 bar, wherein the oxygen partial pressure of 1 to 500 bar being applied to the portion of the liquid solution is reduced to below 5 bar before the portion of the liquid solution is fed back to the rest of the liquid solution.

WO 2016/078698 A1 discloses a method for obtaining a formate from a reaction mixture, in which a polyoxometallate ion, which acts as a catalyst, of the general formula [PMoₓV_{y}O₄₀]ⁿ⁻ is in contact with an organic material at a temperature below 120 °C to produce formic acid in an aqueous solution, wherein 6 ≤ x ≤ 11, 1 ≤ y ≤ 6, x + y = 12 and 3 < n < 10, wherein n, x and y are each an integer. The method comprises the following steps: a) separating a mixture of formic acid and water from the reaction mixture by reverse osmosis and/or as vapor, the vapor subsequently being condensed and b) reacting the formic acid not separated from the water with a hydroxide in aqueous solution to produce a solution of a formate, wherein the organic material is an alpha-hydroxyaldehyde, an alpha-hydroxycarboxylic acid, a carbohydrate, or a glycoside.

The problem to be solved by the present invention is to provide an alternative method for the production of an alkanoic acid. In particular, the method shall enable the degradation of a complex biomass without requirement of its prior disintegration, e.g. by use of a strong organic or inorganic acid.

The problem is solved by the subject-matter of claim 1. Embodiments of the invention are subject-matter of claims 2 to 15.

According to the invention a method for obtaining an alkanoic acid is provided. Molecular oxygen, a solid biogenic material and a solution of a catalyst in a solvent are used in a catalytic reaction. The catalyst is a polyoxometallate ion of the general formula [PMoₓV_{y}O₄₀]ⁿ⁻, wherein 6 ≤ x ≤ 11, 1 ≤ y ≤ 6 and x + y = 12, [WₓV_{y}O₁₉]ⁿ⁻, where x + y = 6, 3 ≤ x ≤ 5 and 1 ≤ y ≤ 3 or [P₂WₓV_{y}O₆₂]ⁿ⁻, where x + y = 18, 12 ≤ x ≤ 17 and 1 ≤ y ≤ 6, or the catalyst is a VO²⁺ containing salt or a [VO₃]⁻ containing salt, wherein n, x and y are each an integer, or the catalyst is a vanadium phosphate, a vanadium pentoxide, an ammonium iron sulfate, a copper sulfate or a nickel sulfate. The biogenic material is insoluble in the solution of the catalyst. The method comprises the steps of
a) providing the molecular oxygen, the biogenic material and the solution of the catalyst,
b) forming a triphasic composition by bringing into contact the biogenic material, the solution of the catalyst and the molecular oxygen, wherein a total dry weight of the biogenic material in relation to a total weight of the biogenic material together with a total weight of the solution of the catalyst is in a range of 15 wt.% to 75 wt.%, wherein a film of the solution of the catalyst is formed on a surface of the biogenic material and/or wherein the biogenic material is moistened and/or soaked with the solution of the catalyst, wherein the film and/or the biogenic material is/are in direct contact with the molecular oxygen,
c) heating the triphasic composition obtained in step b) to a temperature in the range of 70 °C to 200 °C and maintaining the temperature until at least a part, in particular at least 50 wt.% of the total dry weight, of the biogenic material is decomposed, wherein the heating and the maintaining is performed at an oxygen partial pressure in the range of 1 bar to 50 bar.

In contrast to the methods known from WO 2012/034839 A1, WO 2016/116405 A1, EP 3 484 846 A1, WO 2016/120169 A1 and WO 2016/078698 A1, the relation of the total dry weight of the biogenic material to the total weight of the biogenic material together with a total weight of the solution of the catalyst is at least 15 wt.% in the method according to the invention. The total weight of the solid biogenic material is equal to the sum of the total dry weight of the solid biogenic material and the total weight of vaporizable liquid, in particular water, contained in the solid biogenic material. The total dry weight - which term is here synonymously used with the term "total dry matter" - is the total weight of solids in the solid biogenic material remaining after its total drying. The total dry weight of the solid biogenic material can be measured by weighing the solid biogenic material after its total drying. The total weight of vaporizable liquid can be determined by subtraction of the weight of the solid biogenic material after total drying from the weight of the solid biogenic material before total drying. From the weight of a sample of the solid biogenic material before and after total drying, the percentage of the total dry weight from the total weight of the solid biogenic material can be determined. The total dry weight of the solid biogenic material can then be calculated at any time from its weight. The terms "total dry weight" and "total dry matter" are common technical terms in waste technology, paper production, wood processing, food production and/or feed production. The total dry weight of the solid biogenic material may comprise the weight of carbohydrates, chitin, lignin, tannins, fats, proteins, vitamins, minerals and antioxidants contained in the solid biogenic material.

In the known catalytic oxidation reactions the biogenic material is always present in solution or in suspension. In this case the relation of the total dry weight of the biogenic material to the total weight of the biogenic material together with a total weight of the solution of the catalyst does not exceed about 12 wt.%. In case the relation is higher than 12 wt.% the resulting composition cannot be considered as suspension but, e.g., as a lumpy material, i. e. a material in which particles or pieces of the biogenic material cannot freely flow within the solution of the catalyst. Usual processing of the biogenic material, e. g. by pumping or stirring, is difficult in case the relation is higher than 12 wt.% and it becomes the more difficult the higher this relation is. Furthermore, dissolving oxygen in the solution of the catalyst by blowing or mixing oxygen or air into the solution of the catalyst, e. g. by use of a static mixer, is difficult when the relation is higher than 12 wt.%. Therefore, the person skilled in the art would not consider a relation of the total dry weight of the biogenic material to the total weight of the biogenic material together with a total weight of the solution of the catalyst exceeding 12 wt.% for providing an alternative method for the production of an alkanoic acid.

The method according to the invention enables the use of a relatively high portion of solid biomass by weight, in particular solid biomass in a portion of at least 15 wt.%, in particular of at least 20 wt.%, in particular of at least 25 wt.%, in particular of at least 30 wt.%, in particular of at least 35 wt.%, in particular of at least 40 wt.%, and of at most 75 wt.%, in particular of at most 70 wt.%, in particular of at most 65 wt.%, in particular of at most 60 wt.%, in particular of at most 55 wt.%, in particular of at most 50 wt.%, in particular of at most 45 wt.%, of the total quantity of the reaction components by weight. The relatively high portion of solid biomass by weight can be efficiently decomposed in the method according to the invention. Thus, the method according to the invention starts with a wet solid biomass and results in a liquid solution having a relatively high alkanoic acid concentration, in particular an alkanoic acid concentration of up to 45 wt.%, in particular of up to 40 wt.%, in particular of up to 35 wt.%, in particular of up to 30 wt.%, in particular of up to 25 wt.%, in particular of up to 20 wt.%, in particular of up to 15 wt.%.

The inventors of the present invention found that the method according to the invention allows a very effective catalytic oxidation of the solid biogenic material. The inventors further found that the catalytic oxidation becomes the more effective the more the relation of the total dry weight of the biogenic material to the total weight of the biogenic material together with a total weight of the solution of the catalyst exceeds 15 wt.%. It has also been recognized as important for the effectivity that the molecular oxygen, i. e. oxygen in form of an oxygen-containing gas phase, e. g. air, or a gas phase consisting of oxygen, is in direct contact with the film covering the solid biogenic material or with the solid biogenic material soaked or moistened with the solution of the catalyst. The biogenic material covered with the film or soaked or moistened with the solution of the catalyst is usually in direct contact with the molecular oxygen by being surrounded by the molecular oxygen at all sites except sites that are in direct contact with another solid surface or other solid surfaces. This other solid surface or these other solid surfaces may be the surface(s) of other sites of the biogenic material or the wall(s), top or bottom of a vessel in which the triphasic composition is present during reaction or the bottom on which the triphasic composition is present during reaction. At least 70 wt.%, in particular at least 80 wt.%, in particular at least 90 wt.%, in particular at least 95 wt.%, and at most 100 wt.%, in particular at most 99.9 wt.%, of the total weight of the biogenic material may be in direct contact with the molecular oxygen. A direct contact with the molecular oxygen of 100 wt.% of the total weight of the biogenic material may be achieved in a fluidized bed reactor.

The inventors of the present invention further found that the alkanoic acid accumulates in the solution of the catalyst and that the method according to the invention results in an alkanoic acid content in this solution of up to 45 wt.% of the total dry weight of the biogenic material present at the beginning of performance of the method according to the invention. Products resulting from decomposition of the biogenic material are predominantly water and the alkanoic acid. This further distinguishes the method according to the invention from the catalytic oxidation of biogenic materials in solution and/or in suspension known in the art. The method according to the invention allows obtaining an alkanoic acid in a relatively high yield by catalytic oxidation of a solid biogenic material.

The triphasic composition formed at step b) comprises or consists of a solid material in form of the biogenic material, a liquid material in form of the solution of the catalyst, and a gaseous material in form of the molecular oxygen.

The film of the solution of the catalyst on the surface of the biogenic material is to be understood as an interrupted or in particular a continuous liquid layer on the surface of the biogenic material. Usually, this layer remains on the surface even when the solution of the catalyst, in particular an excess solution of the catalyst, is drained off. The film is that part of the solution that is retained on the surface of the biogenic material by intermolecular forces, in particular van der Waals forces, between molecules of the surface of the biogenic material and molecules of the solution of the catalyst. The film has a phase boundary to the molecular oxygen and a further phase boundary to the solid biogenic material. The contact area between the film on the solid biogenic material and the molecular oxygen is usually 100 % of the surface area of the film. The surface-area-to-volume ratio of the solution of the catalyst forming the film is much higher than that of the solution of the catalyst in every suspension of biogenic material known in the art. Since the relatively low volume of the solution of the catalyst means that only a relatively small amount of the catalyst is present in the oxidation reaction, the relatively high efficiency of the oxidation reaction observed by the inventors is very astonishing. As the catalyst is relatively expensive, this is an important aspect of the invention.

The film may be formed on the surface of the biogenic material by spraying or sprinkling the solution of the catalyst on the surface of the biogenic material or by totally wetting the biogenic material with the solution of the catalyst, in particular by immersing the biogenic material in the solution of the catalyst, followed by draining the solution of the catalyst from the biogenic material, in particular the excess solution of the catalyst. Draining the solution of the catalyst, in particular the excess solution of the catalyst, may be performed as an active draining, e. g. by blowing off excess solution from the biogenic material, or as a passive draining, i.e. by letting the solution of the catalyst drain off by gravity.

Drying-out of the film and/or the moistened and/or soaked biogenic material may be prevented by continuously spraying or sprinkling the solution of the catalyst on the surface of the biogenic material, in particular in a trickle-bed reactor, or by enclosing the triphasic composition in an atmosphere saturated with vapor of the solvent.

From the above it is clear that the triphasic composition is not a suspension of the solid biogenic material in the solution of the catalyst and in particular not such a suspension through which molecular oxygen bubbles.

During steps b) and c), in particular during step c), the solid biogenic material is decomposed by catalytic oxidation by means of the catalyst to generate the alkanoic acid. Catalytic oxidation of the solid biogenic material by the catalyst results in the reduction of the catalyst. The reduced catalyst is not able to perform catalytic oxidation of the solid biogenic material. The contact area between the film of the solution of the catalyst on the solid biogenic material and the molecular oxygen is relatively large in relation to the volume of the film. In case of the biogenic material soaked with the solution of the catalyst, the molecular oxygen can penetrate the soaked biogenic material. This also results in a contact area between the solution of the catalyst within the solid biogenic material and the molecular oxygen that is relatively large in relation to the volume of the solution of the catalyst. By the oxygen partial pressure in the range of 1 bar to 50 bar, solving of the molecular oxygen in the solution of the catalyst is promoted. All this together results in a relatively effective mass transfer of the molecular oxygen into the film and/or in the solution of the catalyst within the solid biogenic material and thus in a very effective and continuous oxidation of the reduced catalyst. This allows a continuous and effective production of alkanoic acid by the method according to the invention.

The inventors of the present invention found, that in the method according to the invention, the undesired further catalytic oxidation of the alkanoic acid to CO₂ is relatively low. The reason for this could be that the alkanoic acid formed by decomposition of the biogenic material usually drains off from the remaining biogenic material and collects at the bottom. This results in a much smaller surface-area-to-volume ratio than in the film and thus could result in a much lower oxidative activity in this solution containing the alkanoic acid than in the film.

By obtaining the alkanoic acid by catalytic oxidation of a solid biogenic material not present in a suspension, the method according to the invention differs from the liquid phase processes known from the above prior art documents. In the liquid phase process, a pretreatment of the suspended solid biogenic material may be necessary or at least advantageous for accelerating the catalytic oxidation. The pretreatment may comprise disintegration of cellulose and/or lignin or addition of relatively strong organic acids or inorganic acids, in particular hydrochloric acid and sulfuric acid. The pretreatment mainly removes the hemicellulotic fractions, storage compounds and extractives, such as starch and saccharides, from the solid biogenic material. These hemicellulotic fractions can then be dissolved in the liquid phase and thus oxidized. In order to oxidize the remaining fractions of the biogenic material obtained during pretreatment, in particular cellulose and lignin, in the liquid phase process, further treatment of this material is necessary. In addition, a significant proportion of the cellulose and lignin is discharged after solid biogenic material pretreatment. Therefore, in relation to the carbon content of the biogenic material, only a relatively small proportion of the alkanoic acid produced in this way can be attributed to cellulose and lignin. Examples for pretreatment procedures and further treatment procedures are the Bergius process, the Arkenol^{™} process and biorefining processes, in particular the Organosolv process, the Chempolis^{™} process, the sunliquid^{®} process and the Plantrose^{®} process. However, pretreatment procedures and further treatment procedures will always result in fractions of an educt remaining as constituent or even main constituent in the obtained reaction mixture. Examples of remaining main constituents are cellulose, lignin or wood. The remaining main constituents may remain in a relatively high content of at least 20 wt.% in the obtained reaction mixture. In contrast to that, in the method according to the invention no pretreatment or further treatment is necessary in order to selectively and efficiently convert cellulose or lignin into alkanoic acid, even if the catalyst concentration is relatively low. The method according to the invention enables a conversion of up to 100 wt.% of cellulose and lignin.

The solid biogenic material may be chitin from animals, such as insects or crustaceans, a plant-based solid biogenic material, a sewage sludge or a bioplastic. The plant-based solid biogenic material may be a plant-based solid biogenic material containing lignin, cellulose, hemicellulose and/or chitin, in particular a plant-based solid biogenic material containing lignin, cellulose and/or chitin. The chitin may be chitin from fungi. The sewage sludge may be a sewage sludge containing lignin, cellulose and/or hemicellulose, in particular a sewage sludge containing lignin and/or cellulose. In the context of the present invention, a bioplastic is a plastic derived from a biomass source. For example, a bioplastic may be derived from lactide, glucose, molasses, vegetable oil, starch, in particular corn starch, pea starch, potato starch, or microbiota. The bioplastic may be a bioplastic containing lignin, cellulose and/or hemicellulose, in particular a bioplastic containing lignin and/or cellulose. In particular, the bioplastic may be thermoplastic starch, in particular thermoplastic modified starch or polylactic acid (PLA) which polylactic acid may be derived from lactide obtained from glucose and molasses. The inventors of the present invention found that contrary to catalytic oxidations in known methods lignin, cellulose and hemicellulose are directly catalytically oxidized to alkanoic acid by the method according to the invention. The catalytic oxidation of lignin, cellulose and hemicellulose in the plant-based solid biogenic material may result from a relatively high concentration of the molecular oxygen in the film of the solution of the catalyst and/or in the solution of the catalyst on the moistened plant-based biogenic material and/or within the soaked biogenic material.

The catalytic oxidation of lignin and cellulose can be achieved by the method according to the invention without pre-treatment of the solid biogenic material and without the addition of an additive, in particular without the addition of an acid, a derivative of an acid or a salt of an acid.

With the method according to the invention the inventors achieved an alkanoic acid content of up to 45 wt.% of the total weight of the decomposition products. In case an alkanoic acid content is given in relation to the total weight of the decomposition products, the term "decomposition products" refers here and in the following to the solution or suspension resulting after the decomposition. This solution or suspension contains the solution of the catalyst, all products formed by the catalytic reaction and the residues of the biogenic material remaining after the catalytic reaction. The achievable alkanoic acid content is dependent on the solid biogenic material, the degree of moisture of the solid biogenic material and the ratio of the total dry weight of the solid biogenic material to the total weight of the catalyst. The inventors found that a relatively low degree of moisture results in a relatively high alkanoic acid content in relation to the total weight of the decomposition products. Furthermore, the inventors found that a relatively high ratio of the total dry weight of the solid biogenic material to the total weight of the catalyst results in a relatively high alkanoic acid content in relation to the total weight of the decomposition products. A relatively high alkanoic acid content in the total decomposition products facilitates purification of the alkanoic acid from the decomposition products and makes purification less energy consuming than purification of alkanoic acid less concentrated in the total decomposition products. Thus, the method according to the invention enables a sustainable production of alkanoic acid.

The solid biogenic material may be a material consisting of or comprising an alpha-hydroxyaldehyde, alpha-hydroxycarboxylic acid, carbohydrate, or glycoside, as is known from WO 2012/034839 A1, or polylactic acid. Alpha-hydroxyaldehydes, carbohydrates, and glycosides are present in a large number of renewable resources such as starch, cellulose, or hemicellulose. Starch, cellulose, and hemicellulose are obtained in large quantities as a product of crops or industrial pulping, for example for paper production.

The alpha-hydroxyaldehyde, alpha-hydroxycarboxylic acid, carbohydrate, or glycoside may be a monosaccharide, particularly an aldose, disaccharide, oligosaccharide or polysaccharide, starch, cellulose, hemicellulose, glucose, sucrose, xylose, cellobiose, xylan, heterooligosaccharide, heteropolysaccharide, glycolic acid, polylactic acid or lactic acid, or alpha-hydroxyaldehyde, alpha-hydroxycarboxylic acid, carbohydrate, or glycoside-containing residues or, in particular a renewable, especially an untreated raw material. Untreated means that it has not previously been pulped. The residual material or renewable raw material can be a plant, fungus or bacteria, or components of plants, fungi or bacteria, wood, in particular in the form of wood flour or wood shavings, paper, especially waste paper, algae, cyanobacteria, or silage. The alpha-hydroxyaldehyde, alpha-hydroxycarboxylic acid, carbohydrate, or glycoside may also comprise a mixture of at least two of said substances or have been formed from at least one of said substances or the mixture. The alpha-hydroxyaldehyde, the alpha-hydroxycarboxylic acid, carbohydrate, or glycoside can also comprise a mixture of at least two of the named substances or can be made from at least one of the named substances or the mixture.

Many of the raw materials are obtained as byproducts, for example in paper production, wood processing, food production and feed production. They are thus available as a favorable starting material for the method according to the invention. The method according to the invention can thereby be performed very inexpensively.

The plant-based solid biogenic material may be wood chips, wood pellets, cotton, fungi, sugar beet residues, corn silage, straw, red algae, wheat bran, cardboard, solid digestate, cellulose, a derivative of cellulose, a textile, viscose or a mixture of at least two of wood chips, wood pellets, cotton, fungi, sugar beet residues, corn silage, straw, red algae, wheat bran, cardboard, solid digestate, cellulose, a derivative of cellulose, a textile and viscose. The wood chips may be spruce chips, beech chips, pine chips, in particular pine splint wood chips and pine heartwood chips, and tropical wood chips. The wood pellets may be spruce pellets, beech pellets, pine pellets, in particular pine splint wood pellets and pine heartwood pellets, and tropical wood pellets. The cellulose may be pine cellulose. The derivative of cellulose may be cellulose acetate. The textile may be cotton fabric, hemp fabric, jute fabric, flax fabric, viscose fabric, cellulose acetate fabric or a biodegradable polymer fabric. The bioplastic may be polylactic acid (PLA) or thermoplastic starch, in particular thermoplastic modified starch. The carbon content of the biogenic material is dependent on the biogenic material. For example, the carbon content of spruce chips may be 51 wt.%, the carbon content of beech chips may be 48 wt.%, the carbon content of cotton may be 44 wt.% and the carbon content of straw may be 46 wt.%.

In an embodiment of the invention the maintaining at step c) is performed until at least 50 wt.%, in particular at least 55 wt.%, in particular at least 60 wt.%, in particular at least 65 wt.%, in particular at least 70 wt.%, in particular at least 75 wt.%, in particular at least 80 wt.%, in particular at least 85 wt.%, in particular at least 90 wt.%, in particular at least 95 wt.%, in particular at least 99 wt.%, in particular 100 wt.%, of the total dry weight of the lignin and/or the cellulose is/are decomposed. Decomposition of the total dry weight of the lignin and/or the cellulose can be measured by HPLC or by mass spectrometry of the decomposed material or by the Weender analysis method of the remaining lignin and/or the cellulose. Alternatively, the lignin and/or the cellulose in the solid biogenic material, in particular in a solid polymer, may be hydrolyzed using an acid, in particular sulfuric acid, prior to measurement by HPLC and/or mass spectrometry. In case of cellulose this hydrolysis results in glucose or cellobiose.

The total dry weight of the biogenic material in relation to the total weight of the biogenic material together with the total weight of the solution of the catalyst may be at least 20 wt.%, in particular at least 25 wt.%, in particular at least 30 wt.%, in particular at least 35 wt.%, in particular at least 40 wt.%, in particular at least 45 wt.%, in particular at least 50 wt.%, in particular at least 55 wt.%, and at most 70 wt.%, in particular at most 65 wt.%, in particular at most 60 wt.%.

The VO²⁺ containing salt may be VOSO₄ or vanadyl acetonate. The [VO₃]⁻containing salt may be NH₄VO₃ or sodium metavanadate. The inventors of the present invention found that the VO²⁺ containing salt, in particular VOSO₄, enables a relatively effective catalytic oxidation of the solid biogenic material. The inventors assume that the freely accessible vanadium atoms in dissolved VOSO₄ enable this relatively effective catalytic oxidation of the biogenic material.

The alkanoic acid may be a formic acid or a linear alkanoic acid, in particular acetic acid, or a mixture of at least two different alkanoic acids, in particular a mixture of formic acid and acetic acid.

The solvent in which the catalyst is dissolved may be a polar solvent, i. e. a solvent with a dielectric constant greater than 15. A polar solvent comprises or consists of molecules having a permanent electric dipole moment. The polar solvent may be water, a monohydric alcohol, a trihydric alcohol, dimethyl sulfoxide (DMSO), methylsulfonylmethane or a mixture of at least two of water, a monohydric alcohol, a trihydric alcohol, dimethyl sulfoxide and methylsulfonylmethane. The monohydric alcohol may be methanol, ethanol and propanol. The trihydric alcohol may be glycerin.

The molecular oxygen may be contained in pure oxygen gas or in a gas mixture. The gas mixture may be air or a gas mixture containing a volume percentage of oxygen of at least 10 %, in particular at least 15 %, in particular at least 20 %, in particular at least 25 %, in particular at least 30 %, in particular at least 35 %, in particular at least 40 %, in particular at least 45 %, in particular at least 50 %, in particular at least 55 %, in particular at least 60 %, in particular at least 65 %, in particular at least 70 %, in particular at least 75 %, in particular at least 80 %, in particular at least 85 %, in particular at least 90 %, in particular at least 95 %, in particular at least 99 %. The inventors of the present invention found that a relatively high volume percentage of oxygen, in particular pure oxygen gas, results in a relatively short reaction time and allows the catalytic oxidation reaction to be performed at a relatively low oxygen partial pressure.

The total weight of the solid biogenic material in relation to the total weight of the biogenic material together with a total weight of the solution of the catalyst may be at least 15 wt.%, in particular at least 20 wt.%, in particular at least 25 wt.%, in particular at least 30 wt.%, in particular at least 35 wt.%, in particular at least 40 wt.%, in particular at least 45 wt.%, in particular at least 50 wt.%, in particular at least 55 wt.%, in particular at least 60 wt.%, in particular at least 65 wt.%. The total weight of the solution of the catalyst in relation to the total weight of the biogenic material together with a total weight of the solution of the catalyst may be at most 85 wt.%, in particular at most 80 wt.%, in particular at most 75 wt.%, in particular at most 70 wt.%, in particular at most 65 wt.%, in particular at most 60 wt.%, in particular at most 55 wt.%, in particular at most 50 wt.%, in particular at most 45 wt.%, in particular at most 40 wt.%, in particular at most 35 wt.%.

The total weight of the catalyst in relation to the total dry weight of the biogenic material may be at most 20 wt.%, in particular at most 18 wt.%, in particular at most 16 wt.%, in particular at most 14 wt.%, in particular at most 12 wt.%, in particular at most 6 wt.%, in particular at most 4 wt.%, in particular at most 2 wt.%, in particular at most 1 wt.%, in particular at most 0.5 wt.%.

In the context of the present invention, the solid biogenic material is a material that contains carbon. If the catalyst contains vanadium, the total weight of vanadium contained in the catalyst in relation to the total weight of carbon contained in the solid biogenic material may be at most 6 wt.%, in particular at most 4 wt.%, in particular at most 2 wt.%, in particular at most 1 wt.%. The inventors of the present invention found that a relatively low total weight of vanadium contained in the catalyst in relation to a relatively high total weight of carbon contained in the solid biogenic material allows a relatively efficient catalytic oxidation of the solid biogenic material. The inventors of the present invention further found that a constant total weight of vanadium contained in the catalyst in relation to an increasing total weight of carbon contained in the solid biogenic material results in the obtaining of an increasing amount of alkanoic acid. Thus, a relatively high total weight of carbon contained in the solid biogenic material can be converted without increasing the total weight of the vanadium contained in the catalyst. Therefore, the method according to the invention allows a relatively effective catalytic oxidation with a relatively low total weight content of the catalyst. Due to the relatively high costs of the catalyst, the method according to the invention thus ensures an inexpensive and material-conserving method for obtaining alkanoic acid.

The ratio of the total dry weight of the solid biogenic material to the total weight of the catalyst may be at least 5:1, in particular at least 10:1, in particular at least 20:1, in particular at least 50:1, and at most 170:1, in particular at most 80:1. The inventors of the present invention found that a relatively high ratio of the total dry weight of the solid biogenic material to the total weight of the catalyst results in a relatively effective catalytic oxidation of the solid biogenic material and thus, a relatively high yield of alkanoic acid.

The temperature may be a temperature in the range of 80 °C to 190 °C, in particular a temperature in the range of 85 °C to 185 °C, in particular a temperature in the range of 90 °C to 180 °C, in particular a temperature in the range of 95 °C to 175 °C, in particular a temperature in the range of 100 °C to 170 °C, in particular a temperature in the range of 105 °C to 165 °C, in particular a temperature in the range of 110 °C to 160 °C, in particular a temperature in the range of 115 °C to 155 °C, in particular a temperature in the range of 120 °C to 150 °C, in particular a temperature in the range of 125 °C to 145 °C, in particular a temperature in the range of 130 °C to 140 °C. The temperature may be maintained for at least 30 minutes, in particular at least 1 hour, in particular at least 2 hours, in particular at least 3 hours, in particular at least 4 hours, in particular at least 5 hours, in particular at least 7 hours in particular at least 10 hours, and at most 144 hours, in particular at most 72 hours, in particular at most 48 hours, in particular at most 24 hours, in particular at most 19 hours.

The oxygen partial pressure may be in the range of 1 bar to 24 bar, in particular in the range of 2.1 bar to 9 bar, in particular in the range of 2.5 to 8 bar, in particular in the range of 2,8 bar to 7.5 bar, in particular in the range of 3 bar to 7 bar, in particular in the range of 3.3 bar to 6.5 bar, in particular in the range of 3.7 bar to 6 bar, in particular in the range of 3.8 bar to 5.5 bar; in particular in the range of 3.9 bar to 4.9 bar.

The maintaining according to step c) may be performed until at least 50 wt.%, in particular at least 55 wt.%, in particular at least 60 wt.%, in particular at least 65 wt.%, in particular at least 70 wt.%, in particular at least 75 wt.%, in particular at least 80 wt.%, in particular at least 85 wt.%, in particular at least 90 wt.%, in particular at least 95 wt.%, in particular at least 99 wt.%, in particular 100 wt.%, of the total dry weight of the solid biogenic material is decomposed.

The alkanoic acid produced by the method according to the invention may be separated from the triphasic composition or from the solution of the catalyst by means of a known separation process. The separation process may be by extraction, distillation, filtration or a separation process using a semi-permeable membrane or a combination of at least two of these processes. The semi-permeable membrane may be a membrane permeable for small molecules such as an alkanoic acids, but impermeable for larger molecules such as cellulose or lignin, or a liquid-impermeable, gas-permeable membrane.

During decomposition of the solid biogenic material, CO₂ may be formed as a by-product of catalytic oxidation reaction. The formed CO₂ may be removed, in particular by means of a semi-permeable membrane, in particular a liquid-impermeable, gas-permeable membrane.

The method according to the invention may be performed in a batch reactor. From the batch reactor, a part of the liquid material forming during performance of the method, in particular the solution of the catalyst containing the alkanoic acid and other decomposition products, may be discharged continuously or in intervals, in particular at regular intervals. The alkanoic acid may be separated from the catalyst, in particular by means of distillation or extraction. The remaining solution of the catalyst may be fed back to the triphasic composition, in particular by spraying or sprinkling the remaining solution of the catalyst onto the solid biogenic material, either continuously or at intervals, in particular at regular intervals. This makes it possible to maintain the catalyst used in the process in sufficient amount in contact with the biogenic material during the entire performance of the method. Thus, use of a relatively large amount of the relatively expensive catalyst can be prevented.

The biogenic material may be pre-treated prior to providing the biogenic material according to step a) of the method according to the invention, in particular if the total dry weight of the biogenic material is at most 30 wt.%, in particular at most 25 wt.%, in particular at most 20 wt.%, in particular at most 15 wt.%, in particular at most 10 wt.%, in particular at most 5 wt.%, and if the total vaporizable liquid, in particular water, contained in the biogenic material is at least 70 wt.%, in particular at least 75 wt.%, in particular at least 80 wt.%, in particular at least 85 wt.%, in particular at least 90 wt.%, in particular at least 95 wt.%, of the biogenic material. The pre-treatment of the biogenic material may comprise partial or total drying of the biogenic material. The partial or total drying of the biogenic material increases the total dry weight content of the biogenic material, in particular to a total dry weight content of at least 40 wt.%, in particular at least 45 wt.%, in particular at least 50 wt.%, in particular at least 55 wt.%, in particular at least 60 wt.%, in particular at least 65 wt.%, in particular at least 70 wt.%, in particular at least 75 wt.%, in particular at least 80 wt.%, in particular at least 85 wt.%, in particular at least 90 wt.%, in particular at least 95 wt.%, in particular at least 99 wt.%, and at most 100 wt.%. The inventors of the present invention found that pre-drying the biogenic material to a relatively high total dry weight content of the biogenic material results in the obtaining of alkanoic acid in a relatively high concentration.

The invention will be explained in more detail with reference to the following embodiments.

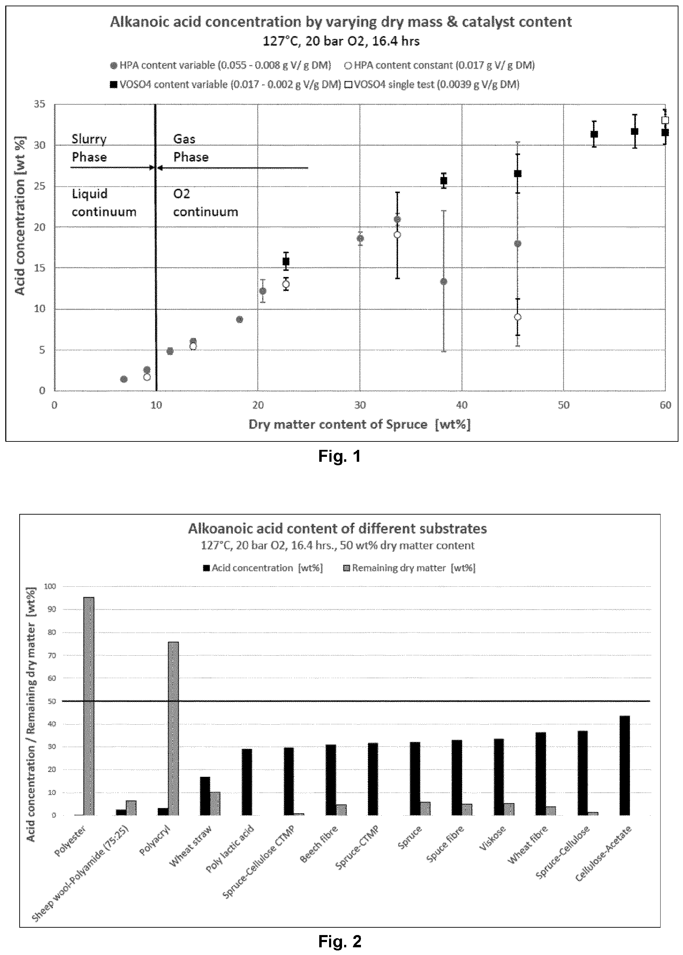

- Fig. 1: shows a graphical representation of the alkanoic acid content in catalytic oxidation reaction mixtures containing spruce chips as a function of the total dry weight content of the spruce chips in the reaction mixture,
- Fig. 2: shows a graphical representation of the alkanoic acid content in catalytic oxidation reaction mixtures containing different solid biogenic materials,

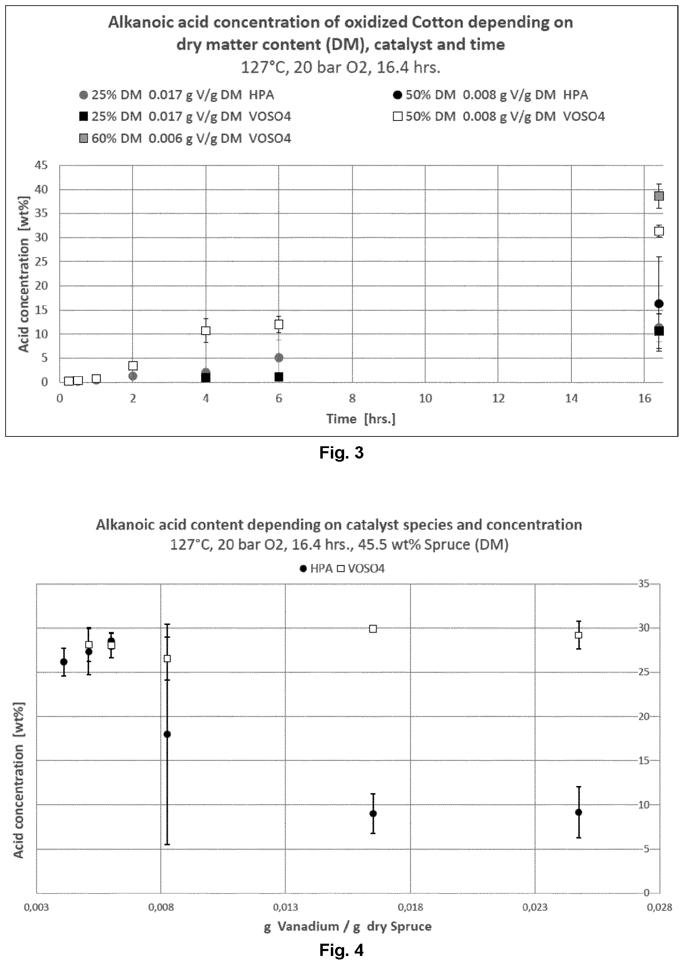

- Fig. 3: shows a graphical representation of the alkanoic acid content in catalytic oxidation reaction mixtures containing cotton linters as a function of the reaction time and the total dry weight content of the cotton linters in the reaction mixture,
- Fig. 4: shows a graphical representation of the alkanoic acid content in catalytic oxidation reaction mixtures containing spruce chips having a total dry weight of 45.5 wt.% of the total weight of the spruce chips together with the total weight of the solution of the catalyst as a function of the ratio of the total weight of the catalytically active vanadium contained in the catalyst to the total dry weight of the spruce chips in the reaction mixture,

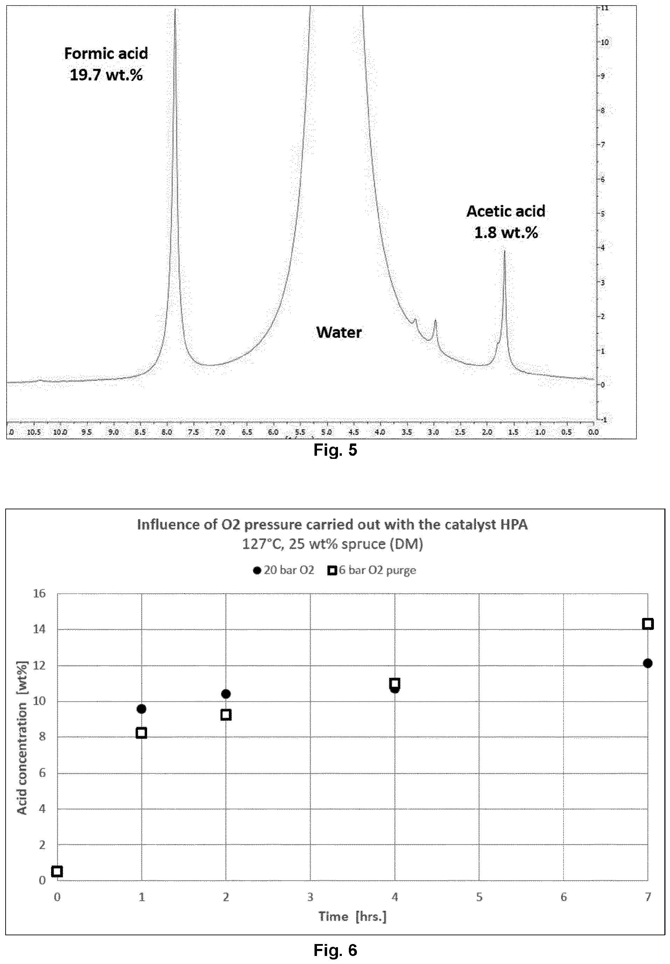

- Fig. 5: shows an ¹H-NMR spectrum of the decomposition products after the catalytic oxidation reaction of spruce chips having a total dry weight of 30 wt.% of the total weight of the spruce chips together with the total weight of the solution of the catalyst,
- Fig. 6: shows a graphical representation of the alkanoic acid content in catalytic oxidation reaction mixtures containing spruce chips having a total dry weight of 25 wt.% of the total weight of the spruce chips together with the total weight of the solution of the catalyst as a function of the reaction time and the oxygen partial pressure,

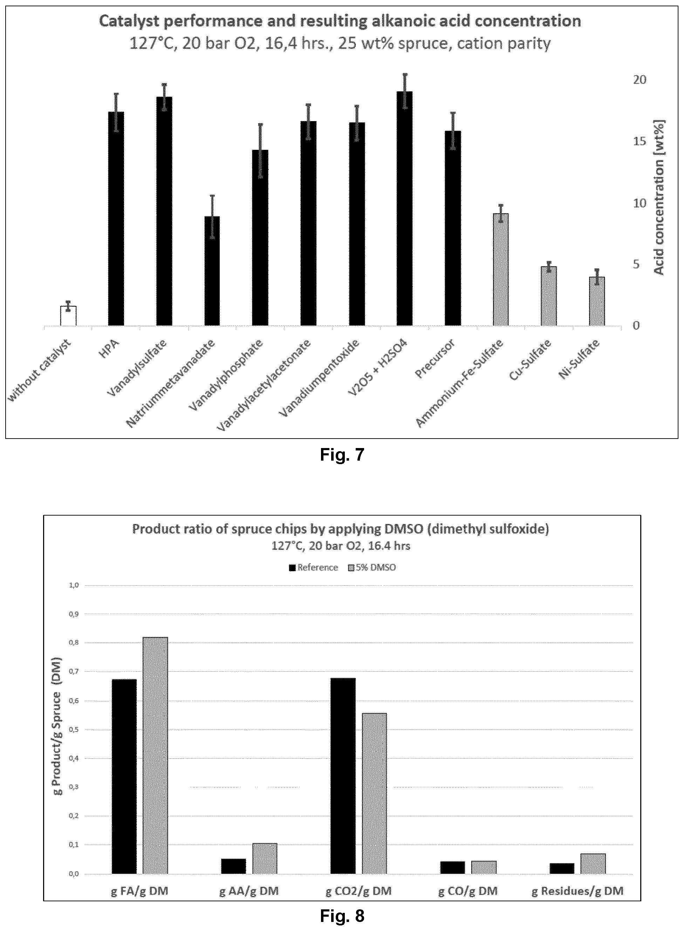

- Fig. 7: shows a graphical representation of the alkanoic acid content in catalytic oxidation reaction mixtures containing spruce chips having a total dry weight of 25 wt.% of the total weight of the spruce chips together with the total weight of the solution of the catalyst using different vanadium-based and vanadium-substituted compounds as catalyst and
- Fig. 8: shows the amounts of different products obtained from catalytic oxidation reaction mixtures containing either DMSO or no DMSO and further containing spruce chips having a total dry weight of 25 wt.% of the total weight of the spruce chips together with the total weight of the solution of the catalyst, wherein the amounts are given in relation to the dry matter content of the spruce chips.

### Example 1: Conversion of spruce chips to formic acid and acetic acid

0.55 g of air-dry spruce chips (90 wt.% total dry weight content/total dry matter content in relation to the total weight of the air-dry spruce chips) are mixed with 1.65 g of a reaction solution containing 1.8 wt.% of a VOSO₄ catalyst in a glass vessel. The mixture is charged into a 10 mL reactor and processed at 127 °C for 16.4 hours at 20 bar O₂. This results in the decomposition of 94 wt.% of the total dry weight of the spruce chips. 15.8 wt.% alkanoic acid content in relation to the total weight of the decomposition products is measured, composed of 15.4 wt.% formic acid and 0.4 wt.% acetic acid. The alkanoic acid determinations were made by titration and ¹H-NMR.

### Example 2: Conversion of cotton cellulose to formic acid

0.55 g of air-dry cotton (95 wt.% total dry weight content/total dry matter content in relation to the total weight of the air-dry cotton) is mixed with 0.37 g of a reaction solution containing 1.8 wt.% of a VOSO₄ catalyst in a glass vessel. The mixture is charged into a 10 mL reactor and processed at 127 °C for 16.4 hours at 20 bar O₂. At the end of the reaction, 95 wt.% of the total dry weight of the cotton is decomposed. 38.9 wt.% alkanoic acid content in relation to the total weight of the decomposition products is measured, composed of 37.7 wt.% formic acid and 1.2 wt.% acetic acid. The alkanoic acid determinations were made by titration and ¹H-NMR.

### Example 3: Conversion of cellulose acetate to acetic acid and formic acid

0.55 g of air-dry cellulose acetate (93 wt.% total dry weight content/total dry matter content in relation to the total weight of the air-dry cellulose acetate) is mixed with 0.55 g of a reaction solution containing 1.8 wt.% of a VOSO₄ catalyst in a glass vessel. The mixture is charged into a 10 mL reactor and processed at 127 °C for 16.4 hours at 20 bar O₂. At the end of the reaction, virtually no solid is present. 43.6 wt.% alkanoic acid content in relation to the total weight of the decomposition products is measured, composed of 17.3 wt.% formic acid and 26.3 wt.% acetic acid. The alkanoic acid determinations were made by titration and ¹H-NMR.

### Example 4: HPA-5-mediated conversion of different feedstocks with a total dry weight content of 25 % in the reaction mixture

Different solid feedstocks are each mixed with a catalyst solution containing 3.5 wt.% of a H₈[PV₅Mo₇O₄₀] (HPA-5) catalyst such that a total dry weight content of 25 wt.% in relation to the total weight of the solid feedstocks together with the total weight of the solution of the catalyst is obtained in the reaction mixture. The mixture is charged into a reactor and processed at 127 °C for 4 hours to 17 hours at 20 bar O₂. At the end of the reaction, the feedstock decomposition rate is measured. Furthermore, the alkanoic acid content in relation to the total weight of the decomposition products is measured by titration and ¹H-NMR.

The results are indicated in Table 1 :

**Table 1: Conversion of different solid feedstocks by HPA-5 catalysis with a total dry weight content of 25 % in the reaction mixture**

| **Substrate** | **Alkanoic acid content (in relation to the total weight of the decomposition products) [wt.%]** | **Decomposition rate in relation to the dry weight of the substrate** |
|---|---|---|
| Spruce chips | 14 | 88 |
| Beech chips | 13 | 98 |
| Pine splint wood chips | 13 | 93 |
| Pine heartwood chips | 11 | 87 |
| Cotton | 11 | 85 |
| Sugar beet | 11 | not determined |
| Corn silage | 7 | 82 |
| Straw | 5 | 88 |
| Red algae | 8 | 92 |
| Tropical wood chips | 5 | 86 |
| Wheat bran | 5 | 97 |
| Cardboard | 4 | 88 |
| Solid digestate | 2 | 81 |

Table 1 shows that the HPA-5-mediated feedstock decomposition rate is in a range of 81 wt.% for solid digestate to 98 wt.% for beech chips. The alkanoic acid content in relation to the total weight of the decomposition products is in a range of 2 wt.% for solid digestate to 14 wt.% for spruce chips. Different feedstocks can be efficiently converted to alkanoic acid by HPA-5-mediated catalytic oxidation.

### Example 5: VOSO₄-mediated conversion of different feedstocks with a total dry weight content of 25 wt.% in the reaction mixture

Different solid feedstocks are each mixed with a catalyst solution containing 1.8 wt.% of a VOSO₄ catalyst such that a total dry weight content of 25 wt.% in relation to the total weight of the solid feedstocks together with the total weight of the solution of the catalyst is obtained in the reaction mixture. The mixture is charged into a reactor and processed at 127 °C for 4 hours to 17 hours at 20 bar O₂. At the end of the reaction, the feedstock decomposition rate is measured. Furthermore, the alkanoic acid content in relation to the total weight of the decomposition products is measured by titration and ¹H-NMR.

The results are indicated in Table 2:

**Table 2: Conversion of different solid feedstocks by VOSO₄ catalysis with a total dry weight content of 25 % in the reaction mixture**

| **Substrate** | **Alkanoic acid content (in relation to the total weight of the decomposition products) [wt.%]** | **Decomposition rate in relation to the dry weight of the substrate** |
|---|---|---|
| Spruce chips | 16 | 94 |
| Beech chips | 15 | 94 |
| Pine splint wood chips | 14 | 92 |
| Pine heartwood chips | 12 | 81 |
| Cotton | 11 | 70 |
| Corn silage | 10 | 85 |
| Corn silage (shredded) | 13 | 74 |
| Straw | 10 | 87 |
| Red algae | 7 | 98 |
| Tropical wood chips | 7 | 66 |
| Solid digestate | 3 | not determined |

Table 2 shows that the VOSO₄-mediated feedstock decomposition rate is in a range of 66 wt.% for tropical wood chips to 98 wt.% for red algae. The alkanoic acid content in relation to the total weight of the decomposition products is in a range of 3 wt.% for solid digestate to 16 wt.% for spruce chips. Different feedstocks can be efficiently converted to alkanoic acid by VOSO₄-mediated catalytic oxidation.

### Example 6: HPA-5-mediated conversion of different feedstocks with a total dry weight content of 50 wt.% in the reaction mixture

Different solid feedstocks are each mixed with a catalyst solution containing 3.5 wt.% of a HPA-5 catalyst such that a total dry weight content of 50 wt.% in relation to the total weight of the solid feedstocks together with the total weight of the solution of the catalyst is obtained in the reaction mixture. The mixture is charged into a reactor and processed at 127 °C for 16.4 hours at 20 bar O₂. At the end of the reaction, the feedstock decomposition rate is measured. Furthermore, the alkanoic acid content in relation to the total weight of the decomposition products is measured by titration and ¹H-NMR.

The results are indicated in Table 3:

**Table 3: Conversion of different solid feedstocks by HPA-5 catalysis with a total dry weight content of 50 % in the reaction mixture**

| **Substrate** | **Alkanoic acid content (in relation to the total weight of the decomposition products) [wt.%]** | **Decomposition rate in relation to the dry weight of the substrate** |
|---|---|---|
| Spruce chips | 27 | 85 |
| Beech chips | 24 | 97 |
| Cotton | 16 | 94 |

Table 3 shows that the HPA-5-mediated feedstock decomposition rate is in a range of 85 wt.% for spruce chips to 97 wt.% for beech chips. The alkanoic acid content in relation to the total weight of the decomposition products is in a range of 16 wt.% for cotton to 27 wt.% for spruce chips. Different feedstocks can be efficiently converted to alkanoic acid by HPA-5-mediated catalytic oxidation.

### Example 7: VOSO₄-mediated conversion of different feedstocks with a total dry weight content of 50 wt.% in the reaction mixture

Different solid feedstocks are each mixed with a catalyst solution containing 1.8 wt.% of a VOSO₄ catalyst such that a total dry weight content of 50 wt.% in relation to the total weight of the solid feedstocks together with the total weight of the solution of the catalyst is obtained in the reaction mixture. The mixture is charged into a reactor and processed at 127 °C for 16.4 hours at 20 bar O₂. At the end of the reaction, the feedstock decomposition rate is measured. Furthermore, the alkanoic acid content in relation to the total weight of the decomposition products is measured by titration and ¹H-NMR.

The results are indicated in Table 4:

**Table 4: Conversion of different solid feedstocks by VOSO₄ catalysis with a total dry weight content of 50 % in the reaction mixture**

| **Substrate** | **Alkanoic acid content (in relation to the total weight of the decomposition products) [wt.%]** | **Decomposition rate in relation to the total dry weight of the substrate** |
|---|---|---|
| Spruce chips | 32 | 94 |
| Cotton | 31 | 96 |
| Pine cellulose | 37 | 99 |
| Viscose | 33 | 95 |
| Cellulose acetate | 44 | > 99 |

Table 4 shows that the VOSO₄-mediated feedstock decomposition rate is in a range of 94 wt.% for spruce chips to more than 99 wt.% for cellulose acetate. The alkanoic acid content in relation to the total weight of the decomposition products is in a range of 31 wt.% for cotton to 44 wt.% for cellulose acetate. Different feedstocks can be efficiently converted to alkanoic acid by VOSO₄-mediated catalytic oxidation. In contrast to the example with HPA-5 as catalyst (example 6), the cotton also shows a relatively high alkanoic acid content. Furthermore, in contrast to the example with HPA-5 as catalyst (example 6), VOSO₄-mediated catalytic oxidation generally results in a relatively high alkanoic acid content.

### Example 8: VOSO₄-mediated conversion of cotton linters with increasing total dry weight contents using an equal amount of catalyst

In separate glass vessels 0.55 g of air-dry cotton linters are each mixed with 1.7 g, 0.6 g and 0.37 g of a catalyst solution containing 1.8 wt.% VOSO₄ catalyst. Thus, the total dry weight content of the mixtures in relation to the total weight of the cotton linters together with the total weight of the solution of the catalyst is 24 wt.%, 48 wt.% and 60 wt.%, respectively, and the total weight of vanadium contained in the catalyst in relation to the total weight of carbon contained in the solid biogenic material is 3.6 wt.%, 1.2 wt.% and 0.8 wt.%, respectively. Each of the mixtures obtained in this way is charged into a 10 mL reactor and processed at 127 °C for 16.4 hours at 20 bar O₂. At the end of the reaction, the alkanoic content in relation to the total weight of the decomposition products is measured by titration and ¹H-NMR.

The mixture initially containing air-dry cotton linters having a total dry weight content of 24 wt.% yields 10.7 wt.% alkanoic acid in relation to the total weight of the decomposition products. The mixture initially containing air-dry cotton linters having a total dry weight content of 48 wt.% yields 31.3 wt.% alkanoic acid in relation to the total weight of the decomposition products. The mixture initially containing air-dry cotton linters having a total dry weight content of 60 wt.% yields 39.0 wt.% alkanoic acid in relation to the total weight of the decomposition products. In all three mixtures, an identical amount of VOSO₄ catalyst in an identical volume of catalyst solution was used. This indicates that a relatively high total dry weight content of the solid biogenic material results in the obtaining of a relatively high total weight of alkanoic acid in a relatively high concentration using a relatively low total weight content of the catalyst. The relatively high concentration of the alkanoic acid in the reaction mixture allows an easy and cost-effective further processing, e.g. by distillation of the reaction mixture in order to separate the alkanoic acid from the reaction mixture.

### Example 9: Catalytic oxidation reaction of spruce chips as a function of the total dry weight content of the spruce chips in the reaction mixture

10 g spruce chips having a total dry weight ranging from 6.8 wt.% to 9.1 wt.% in relation to the total weight of the spruce chips together with the total weight of the solution of the catalyst are each mixed with the catalyst solution containing an HPA-5 catalyst. The obtained mixtures form a slurry and are each charged into a stirred batch autoclave. In these mixtures the spruce chips are surrounded by a liquid continuum.

Furthermore, 0.5 g spruce chips having a total dry weight of at least 11 wt.% and at most 60 wt.% in relation to the total weight of the spruce chips together with the total weight of the solution of the catalyst are each mixed with the catalyst solution containing either an HPA-5 catalyst or a VOSO₄ catalyst. The obtained mixtures are each charged into a 10 mL autoclave without stirring. In these mixtures the spruce chips are covered by a film of the solution the catalyst or moistened or soaked with the solution of the catalyst and surrounded by a gas phase continuum. All the mixtures are processed at 127 °C for 16.4 hours at 20 bar O₂. After the reaction, the alkanoic acid content in relation to the total weight of the decomposition products is measured. The alkanoic acid determinations were made by titration and ¹H-NMR. The results are given in Fig. 1.

Fig. 1 shows the total weight content of alkanoic acid in relation to the total weight of the decomposition products (Acid concentration [wt.%]) obtained in a catalytic oxidation reaction of spruce chips as a function of the total dry weight content of the spruce chips (Dry matter content of Spruce [wt.%]) in the reaction mixture. On the left side of Fig. 1, the results obtained with reaction mixtures processed in the slurry system/liquid continuum with a total dry weight of at most 9.1 wt.% in relation to the total weight of the spruce chips together with the total weight of the solution of the catalyst are shown. On the right side of Fig. 1, the results obtained with reaction mixtures processes in gas phase/O₂ continuum with a total dry weight of at least 11.4 wt.% in relation to the total weight of the spruce chips together with the total weight of the solution of the catalyst are shown.

Furthermore, in some reaction mixtures, the total weight of vanadium contained in the catalyst in relation to the total dry weight of the spruce chips is held constant at 1.7 wt.% and 0.39 wt.% in relation to the total dry weight of the spruce chips for HPA-5 (empty circles) and VOSO₄ (empty square), respectively. In other reaction mixtures, the concentration of the catalyst in the catalyst solution is constant at 1.8 wt.% for VOSO4 and 3.5 wt.% for HPA-5. This results in a total weight of vanadium contained in the catalyst in relation to the total dry weight of the spruce chips is variable ranging from 5.5 wt.% to 0.8 wt.% for HPA-5 (filled circles) and from 1.7 wt.% to 0.2 wt.% for VOSO₄ (filled squares), respectively. In this regard, the total weight of vanadium contained in the catalyst in relation to the total dry weight of the spruce chips decreases with an increasing total dry weight of the spruce chips.

As can be seen in Fig. 1, the total weight content of alkanoic acid obtained from the reaction mixtures in the gas phase/O₂ continuum is higher compared to that obtained from the reaction mixtures in the slurry system/liquid continuum. In the reaction mixtures processed in the gas phase/O₂ continuum, the obtained alkanoic acid content increases with an increasing total dry weight content of the spruce chips in relation to the total weight of the spruce chips together with the total weight of the solution of the catalyst. Interestingly, as can be seen for the reaction mixtures with either constant or variable total weight of vanadium contained in the HPA-5 catalyst in relation to the total dry weight of the spruce chips, a decreasing total weight of vanadium in relation to an increasing total dry weight of the spruce chips results in an increased alkanoic acid content. These results indicate that the reaction mixtures processed in gas phase/O₂ continuum allow the obtaining of a relatively high total weight content of alkanoic acid in reaction mixtures containing a biogenic material having a relatively high total dry weight content. The total weight content of alkanoic acid obtained in the reaction mixtures processed in gas phase/O₂ continuum can be further increased with a decreasing catalyst concentration in relation to an increasing total dry weight content of the biogenic material.

### Example 10: Catalytic oxidation reaction and remaining dry matter of different solid biogenic materials

0.5 g of different dry biogenic materials or dry non-biogenic materials are each mixed with 0.5 g of a catalyst solution containing 1.8 wt.% VOSO₄ such that a total dry weight content of the biogenic material or the non-biogenic material of 50 wt.% in relation to the total weight of the biogenic or non-biogenic material together with the total weight of the solution of the catalyst is obtained in the reaction mixtures. The mixtures are each charged into a 10 mL reactor and processed at 127 °C for 16.4 hours at 20 bar O₂. At the end of the reaction, the remaining dry matter is measured. Furthermore, the alkanoic acid content in relation to the total weight of the decomposition products is measured by titration and ¹H-NMR. The results are given in Fig. 2.

Fig. 2 shows that the remaining dry-matter content is relatively high for the reaction mixtures containing non-biogenic materials, such as polyester and polyacryl. The remaining dry matter content is relatively low for the reaction mixtures containing biogenic materials with a remaining dry matter content of at most 10 wt.%. Furthermore, in contrast to the non-biogenic materials, the biogenic materials are effectively decomposed to a relatively high total weight content of alkanoic acid. These results indicate that the catalytic oxidation reaction is specific for biogenic materials.

### Example 11: Catalytic oxidation reaction of cotton linters as a function of the reaction time and the total dry weight content of the cotton linters

0.53 g cotton linters having a total dry weight / total dry matter (DM) content of 25 wt.%, 50 wt.% and only for VOSO₄ also 60 wt.% in relation to the total weight of the cotton linters together with the total weight of the solution of the catalyst are each mixed with a catalyst solution containing 0.05 g and 0.02 g HPA-5 and 0.03 g, 0.01 g and 0.006 g VOSO₄, respectively. The mixture with cotton linters having a DM content of 25 wt.% contained 0.017 gram vanadium per gram dry matter (g V / g DM) which corresponds to a total weight of vanadium in relation to the total dry weight of the cotton linters of 1.7 wt.%. The mixture with cotton linters having a DM content of 50 wt.% contained 0.008 g V / g DM which corresponds to a total weight of vanadium in relation to the total dry weight of the cotton linters of 0.8 wt.%. The mixture with cotton linters having a DM content of 60 wt.% contained 0.006 g V / g DM, which corresponds to a total weight of vanadium in relation to the total dry weight of the cotton linters of 0.6 wt.%. The obtained mixtures (n = 2-10) are each charged into an 10 mL autoclave and processed at 127 °C until different time-points ranging from 15 minutes to 16.4 hours at 20 bar O₂. After reaching the respective time-point, the alkanoic acid content in relation to the total weight of the decomposition products is measured. The alkanoic acid determinations were made by titration and ¹H-NMR. The results are given in Fig. 3 in which DM means "dry matter content".

Fig. 3 shows the total weight content of alkanoic acid in relation to the total weight of the decomposition products (Acid concentration [wt.%]) obtained in a catalytic oxidation reaction of cotton linters as a function of the reaction time (Time [hours]) and the total dry weight content of the cotton linters in the reaction mixture.

As can be seen in Fig. 3, the total weight content of alkanoic acid obtained from the reaction mixtures increases with an increasing total dry weight content of the cotton linters, despite the reduced total weight of vanadium in relation to the total dry weight of the cotton linters. This effect can be observed after 2 hours and increases with increasing reaction time.

### Example 12: Catalytic oxidation reaction of spruce chips as a function of the ratio of the total weight of the catalytic active vanadium contained in HPA-5 and VOSO₄ catalysts

0.56 g spruce chips having a total dry weight / total dry matter (DM) content of 89 wt.% in relation to the total weight of the spruce chips are each mixed with a reaction solution containing either an HPA-5 catalyst or a VOSO₄ catalyst. In each of the obtained mixtures the total dry weight of the spruce chips is 45.5 wt.% of the total weight of the spruce chips together with the total weight of the solution of the catalyst. The obtained mixtures contained at least 0.004 g V / g DM and at most 0.025 g V / g DM corresponding to a total weight of vanadium in relation to the total dry weight of the spruce chips of at least 0.4 wt.% and at most 2.5 wt.%. The mixtures (n = 5) are each charged into a 10 mL autoclave and processed at 127 °C for 16.4 hours at 20 bar O₂. After the end of the reaction, the alkanoic acid content in relation to the total weight of the decomposition products is measured by titration and ¹H-NMR. The results are given in Fig. 4.

As can be seen in Fig. 4, the total weight content of alkanoic acid obtained from the reaction mixtures containing the HPA-5 catalyst increases with a decreasing total weight of vanadium in relation to the total dry weight of the spruce chips. In the reaction mixtures containing the VOSO₄ catalyst, the total weight of vanadium in relation to the total dry weight of the spruce chips does not affect the total weight content of alkanoic acid obtained which remains in the range of 25 wt.% to 30 wt.%. The inventors assume that the freely accessible vanadium atoms in dissolved VOSO₄ enable a relatively effective catalytic oxidation of the biogenic material even at relatively low total weight of vanadium in relation to the total dry weight of the spruce chips.

### Example 13: ¹H-NMR spectrum of the decomposition products after the catalytic oxidation reaction

0.56 g spruce chips having a total dry weight / total dry matter (DM) content of 89 wt.% in relation to the total weight of the spruce chips are mixed with 1.65 g of a catalyst solution containing 3.51 wt.% HPA-5 catalyst. In the obtained mixture the total dry weight of the spruce chips is 22.6 wt.% of the total weight of the spruce chips together with the total weight of the solution of the catalyst. The mixture is charged into a 10 mL autoclave and processed at 127 °C for 16.4 hours at 20 bar O₂. At the end of the reaction, the decomposition products are measured by ¹H-NMR. The results are given in Fig. 5.

In Fig. 5, the ¹H-NMR spectrum of the decomposition products after the catalytic oxidation reaction is shown. As can be seen in Fig. 5, the decomposition products comprise water, 19.7 wt.% formic acid and 1.8 wt.% acetic acid.

### Example 14: Catalytic oxidation reaction of spruce chips as a function of the reaction time and the oxygen partial pressure

In each of a number of glass vessels 0.55 g air-dry spruce chips having a total dry weight / total dry matter (DM) content of 90 wt.% in relation to the total weight of the spruce chips are mixed with a reaction solution containing 1.45 g of a 1.8 wt.% VOSO₄ catalyst. In each of the obtained mixtures the total dry weight of the spruce chips is 25 wt.% of the total weight of the spruce chips together with the total weight of the solution of the catalyst. The obtained mixtures are each charged into an 10 mL autoclave and processed at 127 °C until different time-points ranging from 20 minutes to 7 hours at either 6 bar O₂ or 20 bar O₂. After reaching the respective time-point, the alkanoic acid content in relation to the total weight of the decomposition products is measured. The alkanoic acid determinations were made by titration and ¹H-NMR. The results are given in Fig. 6.

Fig. 6 shows the content of alkanoic acid c_{Acid} [wt.%] in relation to the total weight of the decomposition products obtained in a catalytic oxidation reaction of spruce chips as a function of the reaction time and the partial oxygen pressure.

As can be seen in Fig. 6, the content of alkanoic acid obtained from the reaction mixtures increases with an increasing reaction time. Furthermore, the partial oxygen pressure does not affect the total weight content of alkanoic acid obtained from the reaction mixtures. Thus, also relatively low partial oxygen pressures can be applied in order to obtain alkanoic acid.

### Example 15: Catalytic activity of different vanadium-based and vanadium-substituted compounds

0.55 g of air-dry spruce chips having a total dry weight / total dry matter (DM) content of 90 wt.% in relation to the total weight of the spruce chips are each mixed with 1.45 g of a catalyst solution containing either a vanadium-based or a vanadium-substituted compound. In each of the obtained mixtures the total dry weight of the spruce chips is 25 wt.% of the total weight of the spruce chips together with the total weight of the solution of the catalyst. The vanadium-based compound is selected from a group consisting of HPA-5, vanadyl sulfate, sodium metavanadate, vanadyl phosphate, vanadyl acetylacetonate, vanadium pentoxide and the HPA-5 precursor H₉PV₁₄O₄₂ (Precursor). The vanadium-substituted compound is selected from a group consisting of ammonium iron sulfate, copper sulfate and nickel sulfate. The catalyst solutions containing the vanadium-based compound each contained 0.007 g of vanadium, respectively. The catalyst solutions containing the vanadium-substituted compound each contained 0.008 g of iron, 0.009 g copper and 0.008 g nickel, respectively. The mixtures are each charged into a 10 mL reactor and processed at 127 °C for 16.4 hours at 20 bar O₂. After the end of the reaction, the alkanoic acid content in relation to the total weight of the decomposition products is measured by titration and ¹H-NMR. The results are given in Fig. 7.

As can be seen in Fig. 7, the reaction mixtures containing the vanadium-based compounds (black bars) yielded a total weight content of alkanoic acid in the range of 8 wt.% to 20 wt.%. The reaction mixtures containing the vanadium-substituted compounds (grey bars) yielded a total weight content of alkanoic acid in the range of 4 wt.% to 10 wt.%. These results indicate that both the vanadium-based compounds and the vanadium-substituted compounds can catalyze the oxidation reaction of biogenic materials in the method according to the invention. However, the vanadium-based compounds show an improved catalytic oxidation.

### Example 16: Catalytic oxidation reaction of polylactic acid with VOSO₄

0.51 g fleece of polylactic acid (PLA) having a total dry weight / total dry matter (DM) content of 99 wt.% in relation to the total weight of the PLA are mixed with 0.485 g of a catalyst solution containing 1.8 wt.% VOSO₄ catalyst. In the obtained mixture the total dry weight of the PLA is 50 wt.% of the total weight of the PLA together with the total weight of the solution of the catalyst. The mixture is charged into a 10 mL autoclave and processed at 127 °C for 16.4 hours at 20 bar O₂. At the end of the reaction, virtually no solid is present. 28.8 wt.% alkanoic acid content in relation to the total weight of the decomposition products is measured. The alkanoic acid is composed of 4.2 wt.% formic acid and 24.6 wt.% acetic acid. The alkanoic acid determinations were made by titration and ¹H-NMR.

### Example 17: Increase of alkanoic acid selectivity by applying DMSO

Two times 0.55 g of spruce chips having a total dry weight / total dry matter (DM) content of 90 wt.% in relation to the total weight of the spruce chips are each mixed with 1.45 g of a catalyst solution containing 1.8 wt.% VOSO₄ catalyst in a vessel. Additionally, 5 wt.% DMSO (in relation to the total weight of the complete reaction mixture including the spruce chips and the catalyst solution) were added to one of the vessels. The obtained mixtures have in each case a dry matter content of 25 wt.% in relation to the total weight of the spruce chips together with the total weight of the solution of the catalyst. The mixtures are charged into a 10 mL autoclave and processed at 127 °C for 16.4 hours at 20 bar O₂. 18.7 wt.% alkanoic acid content in relation to the total weight of the decomposition products is measured for the reference without DSMO and 23.7 wt.% is measured for the reaction in presence of DMSO. The alkanoic acid determinations were made by titration and 1H-NMR.

Figure 8 shows the results in the form of yields given as g product per g total dry matter (DM) of the spruce chips (FA = formic acid; AA = acetic acid). A higher selectivity for alkanoic acid as well as a lower selectivity for CO₂ in case of the DMSO containing assay can be observed. The weight ratio between FA : CO₂ shifts from 1 : 1 (reference without DMSO) to 1.5 : 1 in case of the DMSO containing assay.

### Example 18: Processing with different solvents, mixture and catalysts

0.55 g of spruce chips having a total dry weight / total dry matter (DM) content of 90 wt.% in relation to the total weight of the spruce chips are each mixed with 1.45 g of a catalyst solution in pure water, in pure DMSO, 5 wt.% DMSO (in relation to the total weight of the complete reaction mixture including the spruce chips and the catalyst solution in water) or 5 wt.% methanol (in relation to the total weight of the complete reaction mixture including the spruce chips and the catalyst solution in water). Each catalyst solution was provided with 3.5 wt.% of HPA-5, 2.0 wt.% of VOSO₄ or 1.5 wt.% of NaVOs in relation to the total weight of the spruce chips together with the total weight of the solution of the catalyst. The obtained mixtures have a dry matter content of 25 wt.% in relation to the total weight of the spruce chips together with the total weight of the solution of the catalyst. The mixtures are charged into a 10 mL autoclave and processed at 127 °C for 16.4 hours at 20 bar O₂. The alkanoic acid determinations were made by titration and 1H-NMR.

The highest performances were obtained with VOSO₄ as catalyst. In the assays with pure water an alkanoic acid content in relation to the total weight of the decomposition products of about 18.4 wt.% was measured compared to 11.2 wt.% obtained with HPA-5 and 9.3 wt.% obtained with NaVOs. In cases of pure DMSO, 5 wt.% DMSO and 5 wt.% methanol, nearly all assays resulted in higher acid concentrations. The highest acid contents were achieved with 5 wt.% DMSO which resulted in 23.3 wt.% acid content obtained with VOSO₄, 14.8 wt.% acid content obtained with HPA-5 and 18.2 wt.% acid content obtained with NaVOs. No significant differences in relation to the assay with catalyst solution in pure water were measured for the assay with pure DMSO and HPA-5 and for the assay with 5 wt.% methanol and NaVOs.

## Claims

1. A method for obtaining an alkanoic acid, wherein molecular oxygen, a solid biogenic material and a solution of a catalyst in a solvent are used in a catalytic reaction, wherein the catalyst is a polyoxometallate ion of the general formula [PMoₓV_{y}O₄₀]ⁿ⁻, wherein 6 ≤ x ≤ 11, 1 ≤ y ≤ 6 and x + y = 12, [WₓV_{y}O₁₉]ⁿ⁻, where x + y = 6, 3 ≤ x ≤ 5 and 1 ≤ y ≤ 3 or [P₂WₓV_{y}O₆₂]ⁿ⁻, where x + y = 18, 12 ≤ x ≤ 17 and 1 ≤ y ≤ 6, or wherein the catalyst is a VO²⁺ containing salt or a [VO₃]⁻ containing salt, wherein n, x and y are each an integer, or wherein the catalyst is a vanadium phosphate, a vanadium pentoxide, an ammonium iron sulfate, a copper sulfate or a nickel sulfate, wherein the biogenic material is insoluble in the solution of the catalyst, wherein the method comprises the steps of
a) providing the molecular oxygen, the biogenic material and the solution of the catalyst,
b) forming a triphasic composition by bringing into contact the biogenic material, the solution of the catalyst and the molecular oxygen, wherein a total dry weight of the biogenic material in relation to a total weight of the biogenic material together with a total weight of the solution of the catalyst is in a range of 15 wt.% to 75 wt.%, wherein a film of the solution of the catalyst is formed on a surface of the biogenic material and/or wherein the biogenic material is moistened and/or soaked with the solution of the catalyst, wherein the film and/or the biogenic material is/are in direct contact with the molecular oxygen,
c) heating the triphasic composition obtained in step b) to a temperature in the range of 70 °C to 200 °C and maintaining the temperature until at least a part of the biogenic material is decomposed, wherein the heating and the maintaining is performed at an oxygen partial pressure in the range of 1 bar to 50 bar.

2. Method according to claim 1, wherein the film is formed on the surface of the biogenic material by spraying or sprinkling the solution of the catalyst on the surface of the biogenic material or by totally wetting the biogenic material with the solution of the catalyst, in particular by immersing the biogenic material in the solution of the catalyst, followed by draining the solution of the catalyst from the biogenic material.

3. Method according to claim 1 or 2, wherein drying-out of the film and/or the biogenic material moistened and/or soaked with the solution of the catalyst is prevented by continuously spraying or sprinkling the solution of the catalyst on the surface of the biogenic material, in particular in a trickle-bed reactor, or by enclosing the triphasic composition in an atmosphere saturated with vapor of the solvent.

4. Method according to any of the preceding claims, wherein the biogenic material is chitin from animals, a plant-based biogenic material, a sewage sludge or a bioplastic.

5. Method according to claim 4, wherein the plant-based biogenic material is wood chips, wood pellets, cotton, fungi, sugar beet residues, corn silage, straw, red algae, wheat bran, cardboard, solid digestate, cellulose, a derivative of cellulose, a textile or viscose or a mixture of at least two of wood chips, wood pellets, cotton, fungi, sugar beet residues, corn silage, straw, red algae, wheat bran, cardboard, solid digestate, cellulose, a derivative of cellulose, a textile and viscose, and wherein the bioplastic is polylactic acid (PLA) or thermoplastic starch.

6. Method according to any of the preceding claims, wherein the biogenic material is a biogenic material containing lignin, cellulose and/or chitin.

7. Method according to claim 6, wherein the maintaining at step c) is performed until at least 50 wt.% of the total dry weight of the lignin and/or the cellulose is/are decomposed.

8. Method according to any of the preceding claims, wherein the total dry weight of the biogenic material in relation to the total weight of the biogenic material together with the total weight of the solution of the catalyst is in a range of 20 wt.% to 70 wt.%, in particular in a range of 30 wt.% to 60 wt.%.

9. Method according to any of the preceding claims, wherein the VO²⁺ containing salt is VOSO₄ or vanadyl acetonate and the [VO₃]⁻ containing salt is NH₄VO₃ or sodium metavanadate.

10. Method according to any of the preceding claims, wherein the solution of the catalyst is a solution of the catalyst in water, in a monohydric alcohol, in a trihydric alcohol, in dimethyl sulfoxide (DMSO) or in methylsulfonylmethane or in a mixture of at least two of water, a monohydric alcohol, a trihydric alcohol, DMSO and methylsulfonylmethane.

11. Method according to any of the preceding claims, wherein the molecular oxygen is contained in a gas mixture, in particular air.

12. Method according to any of the preceding claims, wherein the ratio of the total dry weight of the biogenic material to the total weight of the catalyst is at least 5:1, in particular at least 10:1, and at most 170:1, in particular at most 80:1.

13. Method according any of the preceding claims, wherein the temperature is in the range of 80 °C to 190 °C, in particular in the range of 120 °C to 150 °C, and/or wherein the temperature is maintained for at least 30 minutes, in particular at least 2 hours, and at most 144 hours, in particular at most 24 hours.

14. Method according to any of the preceding claims, wherein the oxygen partial pressure is in the range of 2.1 bar to 9 bar, in particular in the range of 2.5 bar to 8 bar.

15. Method according to any of the preceding claims, wherein the maintaining according to step c) is performed until at least 50 wt.%, in particular at least 75 wt.%, of the total dry weight of the biogenic material is decomposed.
